# EUROPEAN PATENT SPECIFICATION

(11) **EP 2 658 984 B1**
(45) Date of publication and mention of the grant of the patent: **30.12.2015**
(21) Application number: 12700002.4
(22) Date of filing: 02.01.2012
(51) Int. Cl.: C12P 7/10, C12P 19/14, C12N 9/42

(54) **EFFICIENT LIGNOCELLULOSE HYDROLYSIS WITH INTEGRATED ENZYME PRODUCTION**
EFFIZIENTE LIGNOCELLULOSEHYDROLYSE MIT INTEGRIERTER ENZYMPRODUKTION
HYDROLYSE EFFICACE DE LA LIGNOCELLULOSE AVEC PRODUCTION D'ENZYMES INTÉGRÉE

(30) Priority: 31.12.2010 EP 10197455
(43) Date of publication of application: 06.11.2013
(73) Proprietor: Clariant Produkte (Deutschland) GmbH, 65929 Frankfurt am Main (DE)
(72) Inventor: RARBACH, Markus, 81477 München (DE); DRAGOVIC, Zdravko, 81375 München (DE); KOHL, Andreas, 81369 München (DE); GERLACH, Jochen, 80687 München (DE); BARTUCH, Jörg, 80687 München (DE); BRÜCK, Thomas, 85452 Eichenried (DE)
(74) Representative: Silber, Anton
(86) International application number: PCT/EP2012/050009
(87) International publication number: WO 2012/089844

(56) References cited:
- WO-A1-2009/049067
- WO-A2-2005/100582
- KOVACS K ET AL: "Enzymatic hydrolysis and simultaneous saccharification and fermentation of steam-pretreated spruce using crude Trichoderma reesei and Trichoderma atroviride enzymes", PROCESS BIOCHEMISTRY, ELSEVIER, NL, vol. 44, no. 12, 1 December 2009 (2009-12-01), pages 1323-1329, XP026708836, ISSN: 1359-5113, DOI: DOI:10.1016/J.PROCBIO.2009.07.006 [retrieved on 2009-07-21]
- KOVACS K ET AL: "Comparative enzymatic hydrolysis of pretreated spruce by supernatants, whole fermentation broths and washed mycelia of Trichoderma reesei and Trichoderma atroviride", BIORESOURCE TECHNOLOGY, ELSEVIER BV, GB, vol. 100, no. 3, 1 February 2009 (2009-02-01), pages 1350-1357, XP025645373, ISSN: 0960-8524, DOI: DOI:10.1016/J.BIORTECH.2008.08.006 [retrieved on 2008-09-14]
- Z. Barta et al: "Process Design and Economics of On-Site cellulase production on Various Carbon Sources in a Softwood-Based Ethanol Plant", Enzyme Research, vol. 2010 2010, 2010, XP002652623, DOI: 10.4061/2010/734182 Retrieved from the Internet: URL:http://www.hindawi.com/journals/er/201 0/734182/cta/ [retrieved on 2011-07-22]

## Description

### Field of the Invention

The present invention provides a process for the biotechnological production of monomeric compounds, such as sugars and/or ethanol, from lignocellulosic feedstocks.

### Background of the Invention

Due to limited resources of mineral oil and demands to reduce CO₂ emissions the chemical industry seeks more sustainable production routes for the manufacture of commodity chemicals such as liquid fuels and base chemicals. Part of that strategy focusses on the conversion of lignocellulosic biomass into versatile chemicals or fuels such as ethanol. Lignocellulosic biomass contains cellulose (∼ 25-40% w/w d.s.), hemicellulose (∼ 15-25% w/w d.s.) and lignin (∼ 15-30% w/w d.s.) as major components and minor amounts of other carbohydrates, waxes, proteins and inorganic compounds. The specific composition of any feedstock may be determined as described by Sluiter et al., 2008. Among forms of plant biomass, lignocellulosic biomass derived from any forestry and agricultural waste streams, such as wood residues and cereal straw are particularly well suited for conversion to commodity chemicals and fuels because of their availability, low cost and environmentally sound production. Additionally, life cycle analyses of production processes utilising lignocellulosic feedstocks indicate reduced greenhouse gas emissions compared to processes based on other feedstocks.

Various process options that describe the conversion of lignocellulosic biomass to ethanol and other base chemicals have been described (Pejo et al., 2008). To realize these processes on an industrial scale it is particularly desirable to transfer the maximal amount of energy, carbon and mass content contained in the renewable feedstock to the end products. At present none of the described conversion processes have realised this to the full extent.

Typical unit operations for the biotechnological conversion of lignocellulosic material (e.g. straw) to value-adding products (e.g. ethanol) are: mechanical de-sizing, physicochemical pre-treatment, enzymatic hydrolysis, fermentation and product recovery.

A key barrier in the realisation of industrial scale cellulosic ethanol production is the cost efficient enzymatic hydrolysis of pre-treated lignocellulose at high solids concentrations.

The hydrolysis of the cellulose fraction has been identified as one of the main obstacles in conversion of lignocellulose to ethanol. At present enzyme cost and performance required for efficient biomass hydrolysis are the major bottlenecks.

Conversion of wood or agricultural lignocellulosic materials into sugars and further to ethanol is a complex process involving several steps comprising sequential combinations of mechanical biomass de-sizing, hydrothermal pre-treatment, enzymatic or chemical biomass hydrolysis, microbial fermentation of biomass hydrolysates and downstream ethanol recovery by distillation or technological equivalents.

### [Mechanical biomass de-sizing]

Typical de-sizing steps involve a mechanical treatment, such as cutting, grinding or milling, of the feedstock, which typically requires significant energy consumption and causes significant operational costs. Feedstocks are cut or ground to particles between 0.5 mm to 2 cm in size, which enables uniform suspension into an aqueous phase. Suspending feedstock particles into a pumpable slurry that can be transferred to downstream unit operations requires large quantities of water, which further adds to operation costs of the process (Tolan, 2002).

### [Pre-treatment]

Conversion of lignocellulosic material to products such as ethanol often comprises a physicochemical pre-treatment step. Pre-treatment aims to remove and separate hemicellulose from cellulose, to disrupt and remove the lignin sheath, to decrease the crystallinity of cellulose, to increase the accessible surface area of cellulose, and/or to increase the pore size of cellulose to facilitate the penetration of hydrolysing agents (Tolan, 2002; Wyman et al., 2005). The pre-treatment step preferentially mobilises the pentose fraction of said biomass, while at the same time it enhances the digestibility of the solid cellulose-containing fraction (Wyman et al., 2005).

The pre-treatment step is often carried out using aqueous slurry. Preferably such slurry has a high solid content, containing a feedstock dry mass in the order of 20 to 40% (w/w). The pre-treatment process often comprises a pressurised hydrothermal treatment (∼ 100-250°C) of the biomass in the absence or presence of acid (i.e. H₂SO₄, HCl, H₃PO₄) or base (i.e. NH₄OH, NaOH, KOH, lime) catalysts, which are added at concentrations between 0.1 and 15% w/w feedstock (Kumar et al., 2009a; Kumar et al., 2009b; Kumar et al., 2009c, Wyman et al., 2005). Reaction times vary between 10s and 2h to provide for efficient transformation of the biomass components in preparation for biomass hydrolysis and fermentation.

Alternative pre-treatment strategies comprise mild hydrothermal treatments combined with the utilisation of organic solvents or ionic liquids to reduce biomass recalcitrance and to solubilise lignin and cellulose components of biomass. These latter pre-treatment options often have higher costs and limited efficiency.

Chandra et al. (2007) describes that hydrothermal pretreatment in the absence or presence of dilute acid is a preferable method to reduce biomass recalcitrance to hydrolysis as it mobilises hemicellulose and partially depolymerises lignin without solubilisation. Additionally it results in amorphous cellulose fibers with a large surface area, which is ideal for enzymatic hydrolysis.

Depending on the nature of the catalysts and the applied temperature profile, pre-treatment can also lead to the formation of soluble inhibitors, including acetic acid, sugar (e.g. furfural, HMF) and lignin degradation products, which can reduce the effectiveness of downstream hydrolysis and fermentation processes (Margeot et al., 2009). To increase the hydrolysis and fermentation effectiveness the pretreatment supernatant needs to be discarded or can be detoxified (Tolan, 2002).

### [Hydrolysis of pre-treated biomass]

Decomposition of the pre-treated biomass slurry into fermentable monomeric sugars can be accomplished by either acid or enzyme catalysed hydrolysis. The enzymatic hydrolysis is more selective and less energy intense than comparable chemical (such as acid-based) methodologies, therefore providing more favourable process economics and potentially a higher ethanol yield during fermentation.

Enzyme systems are mixtures of more than one enzymatic activity. Suitable enzyme systems are in this sense enzyme systems that convert polymeric sugars such as cellulose and hemicellulose into hexose (i.e. glucose) and pentose (i.e. xylose) monomers typically contain cellulase, hemicellulase and beta-glucosidase activities. Enzyme systems containing cellulase and beta-glucosidase activities are often produced in submerged liquid cultures of fungi, e.g. *Trichoderma sp.* and/or *Aspergillus sp..* Residue of fungal biomass is usually separated from the fermentation broth and discarded. The fermentation broth is then concentrated, stabilised and formulated for the resulting enzyme product to be shipped.

Enzymatic biomass hydrolysis is commonly conducted under conditions where total enzyme dosing comprises 1 - 5 % w/w (10-20 FPU/ g cellulose) feedstock. Depending on dosing regime and the specific activity composition of the applied enzyme system, biomass is hydrolysed at 40 - 55 °C for 1 - 7 days. Up to approximately 80-90% w/w of polymeric sugars contained in the biomass are converted into their respective monomers.

According to Kristensen et al. (2009) enzymatic hydrolysis of biomass is often conducted at a lower solids content of 10 - 20 % w/w. A solid content above 15% w/w often leads to significant losses in monomeric sugar yields. This effect is due to problems associated with homogenous mixing of high solid content slurries leading to uneven enzyme distribution. In addition, accumulation of end products like cellobiose and glucose released during enzymatic hydrolysis can lead to inherent inhibition of cellulase and beta-glucosidase activities (Xiao et al., 2004a).

Cardona & Sanchez (2007) describe the use of pre-treatment supernatant and/or previous biomass hydrolysates as growth and enzyme induction substrate for fungal enzyme production. Howard et al. (2003) describe the direct use of spent liquid fermentation broth from fungal fermentation for hydrolysis. Rao et al. (1985) indicate the utilisation of the entire fungal fermentation slurry for efficient hydrolysis of cellulose substrates. Artificial media were used for hydrolysis enzyme production, which does not allow for tailoring of the hydrolysis enzyme systems for a specific feedstock and/or pre-treatment option. Another drawback of the method disclosed by Rao et al. (1985) is that it is limited to secreted enzymatic activities, because nothing is undertaken to facilitate release of non-secretory or cell-surface bound enzymes.

When *Trichoderma*-derived enzyme systems are used for biomass hydrolysis, extracellular beta-glucosidase activities often become rate- and yield-limiting due to their rather low specific activity and significant end product inhibition by glucose released during the process (Xiao et al., 2004a), Shewale, 1982). Therefore, cellulase enzyme preparations or mixtures are typically supplemented with alternative beta-glucosidases (BGL) activities as they are produced for example by *Aspergillus niger* (Seidle et al., 2004). Standard dosing regimes for BGL preparations recommend the addition of 0.01 to 2 Cellobiase units (CBU) per g cellulose to enhance the hydrolysis kinetics and monomeric sugar yields (Chauve et al., 2010).

Alternative strategies to address the technical problem of biomass hydrolysis at higher solid concentrations comprise processes that conduct enzymatic hydrolysis and fermentation simultaneously (SSF).

### [Ethanol Fermentation]

Industrial ethanol production is traditionally carried out by the yeast *S. cerevisiae.* New microbial strains (either yeasts or bacteria) have recently been engineered to efficiently utilize also non-glucose sugars derived from the lignocellulosic raw material. Utilization of pentoses and all hexoses improves the economy of the production of ethanol.

### [Product recovery]

Ethanol is typically recovered from fermentation media by known distillation and/or rectification methods.

### Problem to be solved

Conventional techniques for degradation of biomass are either inefficient or depend on time- and cost-consuming addition of separately produced enzymes or enzyme mixtures which are appropriate for the degradation of the specific biomass. Therefore, the object of the present invention is the provision of an improved process for degradation of biomass, such as lignocellulosic biomass under avoidance of these disadvantages.

### Summary of the invention

The present invention provides a process for degrading pre-treated lignocellulosic biomass comprising the steps:
c) cultivating a microorganism capable of producing at least one enzyme having cellulolytic and/or hemicellulolytic activity in a growth medium, thereby obtaining a microorganism-rich suspension comprising said at least one enzyme;
d) processing the microorganism and/or the micro-organism-rich suspension of step c by
   (i) a mechanical treatment comprising subjection to a volumetric power input of 1-500 kW/m3, for a duration of 0.1-60 min;
      and/or
   (ii) a mechanical treatment which is selected from a treatment with a mixer, a treatment with an homogenizer, and a treatment with a mill;
      and/or
   (iii) a mechanical treatment which involves pumping of the microorganism-containing fermentation slurry from the fermentation vessel to the hydrolysis vessel;
e) subjecting pre-treated lignocellulosic biomass together with the product of step d) to a reactor for biomass hydrolysis to obtain soluble sugars.

Optionally, the pre-treated lignocellulosic biomass has been obtained from lignocellulosic biomass by a physico-chemical treatment. It is possible that the growth medium of step c) comprises lignocellulosic biomass, which has preferably been pre-treated. In a particular embodiment of the above, the final growth medium wherein the microorganism is cultivated comprises part of a pre-treated slurry, so that the process for degrading lignocellulosic biomass comprises the steps:
a) physico-chemical pre-treatment of lignocellulosic biomass to obtain a pre-treated slurry;
b) separating the pre-treated slurry of step a) into two parts, part A and part B;
c) incorporating part A into a raw growth medium to yield a final growth medium, and cultivating at least one microorganism capable of producing at least one enzyme having cellulolytic and/or hemicellulolytic activity in the final growth medium, thereby obtaining a microorganism-rich suspension comprising said at least one enzyme;
d) processing the microorganism and/or the micro-organism-rich suspension of step c , wherein said processing comprises a mechanical treatment which is one or more of the following:
   (i) a mechanical treatment comprising subjection to a volumetric power input of 1-500 kW/m3 for a duration of 0.1-60 min;
      and/or
   (ii) a mechanical treatment which is selected from a treatment with a mixer, a treatment with an homogenizer, and a treatment with a mill;
      and/or
   (iii)a mechanical treatment which involves pumping of the microorganism-containing fermentation slurry from the fermentation vessel to the hydrolysis vessel;
e) subjecting together part B and the product of step d) to a reactor for biomass hydrolysis to obtain soluble sugars.

Fungi are preferred microorganisms in step c). The processing of step d) may occur physically or mechanically or chemically or by a combination thereof. The soluble sugars obtained from step e) may optionally be subjected to downstream conversion processes, such as ethanol production. To that end, a solid/liquid separation may be carried out, such as to obtain a sugar-rich liquid phase comprising the soluble sugars.

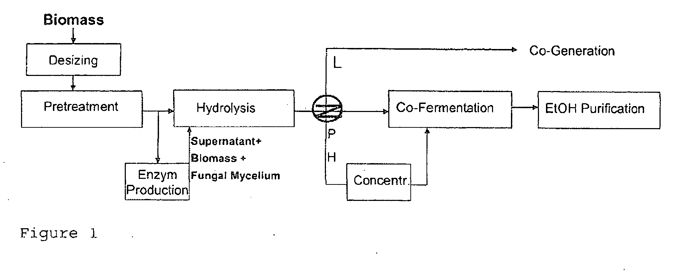

The present invention provides an improved process for hydrolysis of lignocellulosic biomass. The current invention describes a process hydrolysis and fermentation for the production of ethanol from lignocellulosic biomass residues (an example of which is shown in Fig. 1), which integrates the efficient utilisation of integrated enzyme production.

According to the present invention, the production of hydrolysis enzymes is integrated into the process, preferably in close proximity to the hydrolysis process. Subsequently the entire fermentation slurry containing soluble enzyme systems and fungal biomass is used to hydrolyse lignocellulosic feedstocks into component sugars. The integrated production of said enzyme systems using a portion of the pre-treatment feedstock as fermentation medium provides for optimal flexibility in feedstock and pre-treatment options.

In a key aspect of the invention it was surprisingly found that physically or mechanically or chemically processing the microbial biomass, in particular mechanically shearing the fungal biomass, prior to enzyme hydrolysis will result in faster hydrolysis kinetics and superior monomeric sugar (e.g. glucose) yields compared to using the fermentation supernatant or broth without processing the microbial biomass or by using only soluble hydrolysis enzyme components contained in the clarified supernatant of the fermentation broth.

### Detailed description of the invention

The present invention covers an improved process for the degradation of biomass, such as lignocellulosic biomass. The biomass which is subjected to the process according to the present invention may be any biomass as described below. Included therein is biomass comprising large particles or chunks, such as wood residue, corn stover, sugar cane bagasse cereal straw, and therefore mechanical de-sizing may be optionally carried out prior to the actual process of the present invention.

Alternatively, the majority of the biomass particles, such as 90 % or more of the particles, are small enough to be treated in a suspension (slurry), i.e. they have a diameter of no larger than 2 cm, preferentially no larger than 0.5 cm, more preferably no larger than 2 mm, so that no mechanical de-sizing is required. However, in the case that mechanical de-sizing is carried out, it is carried out as follows:

### [Mechanical de-sizing]

According to a preferred embodiment of the invention, any lignocellulosic biomass will be subjected to a coarse de-sizing utilising an apparatus such as a hammer or ball mill or grinder or any type or combinations of such apparatuses suitable to cut the biomass residue to pieces wherein 90 % or more of the particles by mass have a diameter no larger than 2 mm, resulting in small-particle size biomass.

### [Pre-treatment]

The goal of the pre-treatment is a destabilization and/or partial hydrolysis of the polymeric structures of the (small-particle) biomass. The biomass, which is to be subjected to the pre-treatment, is typically dry, i.e. not suspended in a liquid phase. This biomass is then transferred into a reaction vessel using any transportation system known in the art, such as for example a conveyor belt or screw type transporter. The small-particle biomass is transferred to a pressure-resistant vessel and then subjected to a pre-treatment.

The product of the pre-treatment step is synonymously called "slurry" or "pre-treated biomass" or "pre-treated lignocellulosic biomass" or "pre-treated biomass slurry".

The pre-treatment may be a chemical pre-treatment, i.e. a treatment with an acid or base. Preferably, it may be carried out in the presence of an acid catalyst, which in a non-limiting example may be selected from a choice of H₂SO₄, H₂NO₃, HCl, H₃PO₄, SO₂. In a particularly preferred embodiment the acid constitutes H₂SO₄. The acid catalyst may be applied in concentrations of 0-10% w/w d.s. feedstock. In a more preferred embodiment of the invention the acid concentration is adjusted to 0.1-3% w/w d.s. feedstock.

The pre-treatment may alternatively be a thermal treatment, such as exposure to temperatures of more than 80 °C. However, it is most preferred that chemical and thermal treatment be combined. In an even more preferred embodiment of the invention the hydrothermal pre-treatment can either be carried out at or above atmospheric pressure. The pre-treatment in the presence of the acid catalyst may also constitute the application of hot pressurized steam producing temperatures between 120 - 250°C. Under these conditions the biomass may be pre-treated between 0.1-60 min prior to further processing of the biomass and an optional neutralisation step, which may constitute the application of lime or any other base such that the reaction medium has a pH of 3.5 - 6.

In another aspect of the invention de-sizing and pre-treatment may be carried out simultaneously: There, the preferentially dry biomass is transported to the pre-treatment vessel and at the same time contacted with water. The hydrothermal pre-treatment can be mechanically linked to the biomass de-sizing step such that pre-treatment can either be carried out in a batch or continuous operation mode. As a non-limiting example for a continuous pre-treatment mode, the pre-treatment could be carried out in a closed pressure resistant vessel fitted with a screw type conveyor mechanism.

In another preferred embodiment of the invention the hydrothermal pre-treatment is carried out in a steam explosion mode, where biomass is subjected to hot steam injection above atmospheric pressure. The steam induced pressure will preferentially be 1-30 atm (120-250°C) to which said biomass will be exposed for 0.1-60 min. Thereafter, the pressure is suddenly released from the reaction vessel, thereby transferring the pre-treated biomass to a secondary collection vessel. A non-limiting example of a reaction vessel for carrying out steam explosion pre-treatment may be a steam gun reactor (Autoclave Engineers, Erie, Pa) consisting of a steam-jacketed reactor consisting of Hastelloy pipe closed by two ball valves. Associated electrical heaters are placed on all exposed, non-jacketed surfaces of the reactor and controlled to the pre-treatment set point temperature. Direct steam injection is also used to rapidly bring biomass up to pre-treatment temperature. Steam pressure is adjusted and controlled to maintain the desired pre-treatment temperature. All pre-treatment materials exits through a replaceable die at the bottom of the reactor and is collected in a nylon bag supported within a heavy walled, jacketed and cooled flash tank.

It is preferred that processing time and temperature will be selected in a way such that a maximal amount of biomass constituents are hydrolysed to their component monomers in a most efficient and economical way without the production of significant amounts of degradation products such as furfural.

Commonly the lignin fraction of pre-treated feedstocks, will be in the range of 10-70 % w lignin/w d.s. pre-treated feedstock. The lignin content of the pre-treated feedstock can be measured according to methods known to those skilled in the art, such as for example, but not limited to, those disclosed at:
http://www.nrel.gov/biomass/analytical procedures.html.

After the said pre-treatment options the dry solid content of the resulting biomass slurry will be around 10-50% w/w d.s. feedstock. The primary aim of the selected pre-treatment strategy is to select the most energy and cost efficient method that will prepare the lignocellulosic biomass in a way that most of the pentose fraction is solubilised and cellulose fibres will be accessible for enzyme systems utilised in downstream hydrolysis processes. Transfer of the biomass to the specific unit operations will be accomplished by pumping or solely by gravity flow.

### [Core process of the present invention]

The present invention provides a process for degrading pre-treated lignocellulosic biomass comprising the steps:
c) cultivating a microorganism capable of producing at least one enzyme having cellulolytic and/or hemicellulolytic activity in a growth medium, thereby obtaining a microorganism-rich suspension comprising said at least one enzyme;
d) processing the microorganism and/or the micro-organism-rich suspension of step c by
   (i) a mechanical treatment comprising subjection to a volumetric power input of 1-500 kW/m3, for a duration of 0.1-60 min;
      and/or
   (ii) a mechanical treatment which is selected from a treatment with a mixer, a treatment with an homogenizer, and a treatment with a mill;
      and/or
   (iii) a mechanical treatment which involves pumping of the microorganism-containing fermentation slurry from the fermentation vessel to the hydrolysis vessel;
e) subjecting pre-treated lignocellulosic biomass together with the product of step d) to a rector for biomass hydrolysis to obtain soluble sugars.

It is preferred, that in this process the pre-treated lignocellulosic biomass has been obtained from lignocellulosic biomass by a physico-chemical treatment, such as the pre-treatment described above.

In a particularly preferred embodiment of the invention the growth medium of step c) comprises lignocellulosic biomass, which has preferably been pre-treated. Various enzymatic activities, such as - but not limited to - cellulolytic and hemicellulolytic activities, together form an enzyme system, i.e. enzyme systems are mixtures of more than one enzymatic activity. More than one of these activities may in some cases be contained within the same polypeptide molecule, however, it is more typical that an enzyme system comprises a mixture of several, such as more than two, i.e. more than three, different enzymatic polypeptides, wherein each has at least one desired activity, so that the combined activities form the enzyme system.

The processes and systems described by this invention are distinguished from the teachings of prior art. The present invention provides integrated enzyme production, where subsequently pre-treated biomass and the microorganism suspension obtained in step d) are utilised for biomass hydrolysis. It could surprisingly be shown that the presence of the pre-treatment supernatant did not affect downstream enzymatic hydrolysis or fermentation procedures. Further it was surprisingly shown that the use of the entire fermentation slurry comprising mycelium and supernatant from enzyme production showed superior performance in biomass hydrolysis compared to the sole fermentation supernatant obtained from said enzyme production, or compared to comparable commercial enzyme preparations. Further, it could be demonstrated that physically and /or mechanically and/ or chemically processing the microorganism according to step d provides for optimal activation of extra- and intracellular hydrolysis enzyme systems which act synergistically in downstream hydrolysis operations to result in optimal degradation of lignocellulosic feedstocks into monomeric sugar components, such as glucose and xylose.

### [Enzyme production (step c)]

The enzyme production according to step (c) is characterized as follows: pre-treated lignocellulosic biomass slurry is in a first step inoculated with a microorganism, which may be a bacterial, archaeal, yeast or fungal microorganism, which is capable of producing cellulolytic and/or hemicellulolytic enzymatic activities. Cellulolytic activity is an activity capable of degrading, such as hydrolyzing, cellulose, such as being capable of hydrolyzing some or all of the glycosidic bonds therein. Hemicellulolytic activity is an activity capable of degrading, such as hydrolyzing, hemicellulose, such as being capable of hydrolyzing some or all of the glycosidic bonds therein. Hemicellulose includes building blocks such as xylan, glucuronoxylan, arabinoxylan, glucomannan and xyloglucan.

In a non-limiting example said enzymatic activities comprise exo- and endocellulases (i.e. Cellobiohydrolase (CBH) I, II, endoglucanase (EG) I-IV, beta-Glucosidase (BGL)), exo- and endohemicellulases (i.e. xylanase, xylosidase, xylobiase, arabinase, arabinofucosidase, mannanase, mannosidase, galactase and galactosidase) and esterases (Howard et al., 2003, Lynd et al., 2002). Thus, the invention comprises a preferred embodiment wherein the at least one enzyme with cellulolytic and/or hemicellulolytic activity has one or more activities selected from the group consisting of: Cellobiohydrolase type I or type II (CBH I or CBH II), endoglucanase type I, II, III or IV (EGI, EGII, EGIII, EGIV), beta-glucosidase (BGL), esterase, exo-hemicellulase and endo-hemicellulase. Even more preferred is that the exo-hemicellulase and endo-hemicellulase are preferentially selected from xylanase, xylosidase, xylobiase, arabinase, arabinofucosidase, mannanase, mannosidase, galactase and galactosidase.

Non limiting examples for bacteria producing said enzymes activities and which may therefore preferentially be used according to the present invention comprise *Actinobacter* sp., *Agrobacterium* sp., *Bacillus* sp., *Burkholdria* sp., *Clostridia* sp., *Caldicellulosiruptor* sp., *Cellvibrio* sp., *Halobacterium* sp., *Pseudomonas* sp., *Paenibacillus* sp., *Xanthomonas* sp. and *Thermobifida* sp. (Howard et al. 2003, Maki et al., 2009).

Non limiting examples for archaea producing said enzyme activities and which may therefore preferentially be used according to the present invention comprise *Pyrochoccus* sp., *Sulphobolus* sp., *Staphylothermus* sp. and *Thermococcus* sp., (Maki et al., 2009).

Fungi are generally the most preferred microorganisms for the present invention. The process according to this invention may preferentially carried out such that the microorganism is a fungus, which is preferentially selected from the group consisting of: *Trichoderma sp.,* Aspergillus sp., *Penicillium* sp. and *Talaromyces sp.* Non limiting examples for fungi producing said enzymes activities and which may therefore preferentially be used according to the present invention comprise *Aspergillus* sp., *Chaetomium* sp., *Chrysosporium* sp., *Fusarium* sp., *Humicola* sp., *Orpinomyces* sp., *Pencillium* sp., *Phanerochaete* sp., *Piromyces* sp., *Talaromyces* sp., *Trametes* sp. and *Trichoderma* sp. or their respective holomorphs (Howard et al., 2003). Especially preferred is a fungus selected from *Trichoderma* sp. and *Talaromyces* sp.

The choice of the enzyme producing organism can be determined by the carbohydrate composition of the pre-treated feedstock slurry, the amount of the enzyme activities produced by the particular organisms and the biophysical characteristics encompassing parameters such as temperature stability, substrate selectivity, specific activity and inhibitor tolerance.

For the induction of enzyme production said micro-organisms will be incubated at a convenient growth temperature, which can typically vary between 14 and 102 °C, preferably between 28 - 102°C, depending on the microorganism used. Incubation time until onset of enzyme production may vary significantly with the type of micro-organism (Lynd et al., 2002) and pretreated feedstock but can be tested with methods described by Xiao et al. (2004b) or Bobey and Ederer (1981).

In a preferred aspect of the present invention, the enzyme production is achieved with a cellulase hyperproducing strain of the filamentous fungus *Trichoderma reesei* (anamorph: *Hypocrea jecornia*). A non-limiting example of such an organism is the *Trichoderma ressei* strain Rut-C30 (Lynd et al., 2002, Szijarto et al., 2004). In yet another preferred aspect of the invention fungal enzyme systems are produced on pre-treated biomass as the only significant carbon source. This is preferably done with fungi such as *Trichoderma reesei.* Starter cultures of *Trichoderma reesei* can be grown on pre-treated feedstock slurry until spore production commences, which can be quantified using methods such as spectrophotometric measurements at OD₆₀₀ nm. The fungus is then grown for 3 - 7 days under constant mixing at 50-250rpm and pH, temperature as well as oxygen controlled conditions. Throughout the fermentation it is critical that the fungus is incubated at 30°C and at a pH∼5 to yield a maximum amount of enzyme and biomass. At day 5 the maximal biomass and enzyme production is typically accomplished, enabling further processing.

The growth medium typically comprises a carbon source, a nitrogen source, optionally vitamins and trace minerals. The carbon source is supplied at 1-30% w/v, more preferably 1-10% w/v and even more preferably 1-8% w/v to the cultivation medium. Carbon sources may comprise native or pretreated lignocellulosic biomass such as wood, cereal straw, bagasse, switch grass and cellulose, and raw paper pulp obtained from pulp and paper production. Alternative carbon sources may comprise purified cellulose, pulp, milk whey, molasses or sugars such as glucose and lactose. A combination of carbon sources may be used, wherein one carbon source is the product of step b) or an equivalent thereof, whereas a further carbon source, such as one described above in this paragraph is present. The nitrogen source is supplied at 0.1-10% w/v, more preferably 0.1-8% w/v and even more preferably 0.1-4% w/v to the cultivation medium. Nitrogen sources comprise soybean expeller, corn steep liquor, yeast extract, green meal (milled grass), wheat bran, distillers spent grain and inorganic ammonium salts. Vitamins and trace minerals may be supplied at 0-5 % w/v more preferably, 0.001-1% w/v to the cultivation medium. Trace minerals supplied to the cultivation medium may comprise Fe, Mn, Zn and Co salts, while vitamins added to the cultivation medium may comprise Vitamin B complexes, biotin, coalbumin.

Exact cultivation media compositions and physical conditions for effective growth of cellulase enzyme producers are known to those skilled in the art. The final selection of the growth medium will depend on the microorganism used.

In a preferred embodiment of the invention both the microorganism biomass and the spent fermentation medium containing residual lignocellulosic biomass and the majority of the cellulase and hemicellulase enzyme activities will be used in the downstream biomass processing step.

In the present invention it was surprisingly found that significant proportions of the cellulase and hemicellulase activities are retained on residual pre-treated feedstock biomass and produced fungal biomass.

Even more interestingly it could be demonstrated that biomass hydrolysis was more efficient when the entire enzyme containing fermentation slurry (supernatant and mycelium/ biomass) was applied as compared to hydrolysis experiments applying only enzyme-containing fermentation supernatant or commercial enzyme preparations.

This may optionally be achieved by splitting the biomass slurry obtainable by the pre-treatment into two streams, such that one part is subjected to step c), while another part is directly subjected to step e). The investigators have surprisingly found that part of the pre-treated biomass can be subjected to the growth medium of the microorganism. It is understood that the microorganism is thereby capable of producing enzymes suitable for degradation of the pre-treated biomass. Thus the present invention also provides within a preferred embodiment a process for degrading lignocellulosic biomass comprising the steps:
a) physico-chemical pre-treatment of lignocellulosic biomass to obtain a pre-treated slurry;
b) separating the pre-treated slurry of step a) into two parts, part A and part B;
c) incorporating part A into a raw growth medium to yield a final growth medium, and cultivating at least one microorganism capable of producing at least one enzyme having cellulolytic and/or hemicellulolytic activity in the final growth medium, thereby obtaining a microorganism-rich suspension comprising said at least one enzyme;
d) processing the microorganism and/or the micro-organism-rich suspension of step c , wherein said processing comprises a mechanical treatment which is one or more of the following:
   (i) a mechanical treatment comprising subjection to a volumetric power input of 1-500 kW/m3 for a duration of 0.1-60 min;
      and/or
   (ii) a mechanical treatment which is selected from a treatment with a mixer, a treatment with an homogenizer, and a treatment with a mill;
      and/or
   (iii)a mechanical treatment which involves pumping of the microorganism-containing fermentation slurry from the fermentation vessel to the hydrolysis vessel;
e) subjecting together part B and the product of step d) to a reactor for biomass hydrolysis to obtain soluble sugars.

A flow-chart of the process is given in Figure 1.

In a preferred embodiment of this process part A is the minor part of the pre-treated slurry obtained in step a, preferably 1 to 20 % (weight dry solids), more preferably 1 to 5 % (weight dry solids) and most preferably 1 to 5 % (weight dry solids). Solids are rigid particles, i.e. particles which are not solubilised in the liquid phase at the process conditions used.

### [Processing of the microorganism (step (d)]

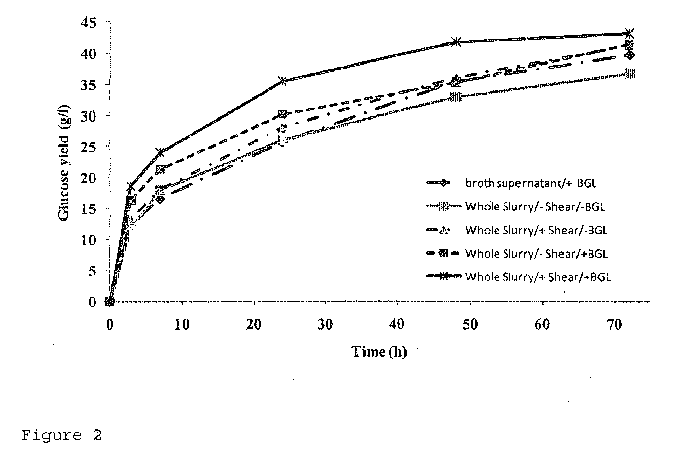

It was surprisingly found that physically and/or mechanically and/or chemically processing the fermentation slurry in step d) prior to the hydrolysis step e) and subjecting the processed product to step e) will enhance biomass hydrolysis kinetics and leads to higher soluble sugar yields (Figure 2). As higher yield of soluble sugar is thereby understood when the yield which can be obtained after 72 h under the conditions described in example 4 is higher when the product of step c) is subjected to step d) than when step d) is omitted. The prior art (Rao et al., 1985) fails to disclose such a treatment. Control experiments, where a commercial cellulase (Celluclast, Novozymes, Denkmark) / BGL (Novo 188, Novozymes, Denkmark) preparation was mechanically processed prior to lignocelluloses hydrolysis did not result in an enhancement of lignocellulose hydrolysis kinetics or a higher soluble sugar yield.

In one embodiment, the microorganism obtained by the culturing step c) is isolated after the culturing step, such as for example by centrifugation or other procedures well known in the art. In this embodiment, the isolated microorganism is subjected to step d). However, in an alternative and more preferred embodiment of the invention, the entire microorganism-containing suspension, i.e. the culture product of (c) comprising the microorganism and the growth medium, is physically and/or mechanically and/or chemically processed prior to biomass hydrolysis. Utilisation of the entire microorganism-containing suspension and not only the supernatant of the microorganism-containing suspension for biomass hydrolysis allows for a more efficient enzyme dosing in downstream biomass hydrolysis operations, as the investigators of this invention have surprisingly found out. The treatment may be physical, mechanical, chemical, or a combination thereof.

In a preferred practice of the invention, the microorganism-containing suspension is subjected to stirring at increased rotor speeds in the fermentation vessel. In a yet another preferred practice of the invention the microorganism-containing suspension is pumped and sheared in a transfer pipe containing an ultrathorax. Either of these embodiments may be particularly useful if the microorganism is a fungus, so that the treatment comprises a treatment of the fungal mycelium.

The mechanical treatment may be carried out as follows: It may comprise subjection of the microorganism or the microorganism suspension to a volumetric power input (also termed "power input") of 1 - 500 kW/m³, more preferably 1 - 200 kW/m³, even more preferably 1 - 100 kW/m³, preferably for a duration of 0.1 - 60 min, more preferably 1 - 30 min, and even more preferably 1 - 10 min.

The mechanical treatment may be selected from a treatment with a mixer, a treatment with a homogenizer, and a treatment with a mill. Such mechanical treatment can add mechanical shearing stress or grinding force to the microorganism and can disrupt or destroy the cell membranes and/or the cell walls, or the fungal mycelium or parts thereof. More than one of such treatments may be combined with each other. The rupture or destruction of the cell structures, such as cell membranes and/or cell walls, or of the fungal mycelium or parts thereof, can be tested by methods well known by the person skilled in the art, such as microscopy.

In a preferred practice of this invention the microorganism-containing suspension is treated, i.e. sheared by increasing the impellor speed at the end of the fermentative enzyme production cycle.

The effectiveness of the mechanical process is controlled by the relative power gradient applied to the microorganism biomass in a reaction vessel.

Alternatively the shear stress, which leads to an increase in hydrolysis enzyme activity, may be induced by pumping the microorganism-containing fermentation slurry from the fermentation to the hydrolysis vessel. The microorganism-containing slurry may be pumped and sheared in a transfer pipe. The microorganism may thereby be sheared. In terms of shearing rates, the microorganism containing slurry is subjected to 1600 - 50000 l/s, more preferably 1600 - 27000 l/s, and even more preferably 1600 - 10000 l/s. This treatment is suitable to induce an increase in hydrolysis enzyme activity. This treatment may be done preferably for a period of 0.01 to 100 s.

Alternatively the microorganism-containing slurry may be subjected to high pressure homogenisation or to ultrasonic treatment or to other devices known by the person skilled in the art to induce high shear stress such as an Ultraturax. In a preferred practice of the invention, the microorganism-containing slurry is subjected to stirring at increased rotor speeds in the fermentation vessel. In a yet another preferred practice of the invention the microorganism-containing suspension is pumped in a transfer pipe. The microorganism may thereby be sheared. In terms of shearing rates the microorganism-containing slurry is subjected to 1600 - 50000 l/s, more preferably 1600 - 27000 l/s, and even more preferably 1600 - 10000 l/s to induce an increase in hydrolysis enzyme activity for a period of 0.01 to 100 s.

The reader will understand that the invention comprises any specific combination of the values disclosed herein. For example, any specific shearing rate or range of shearing rate disclosed herein is to be understood as specifically disclosed in combination with any specific time for carrying out such treatment (min or s) disclosed herein. Also, any volumetric power input or range of volumetric power input disclosed herein is to be understood as specifically disclosed in combination with any specific time for applying such input (min or s) disclosed herein. Also part of the invention are those embodiments, wherein the product of volumetric power input (kW/m³) and time (min) is equivalent to any product thereof disclosed herein. Also part of the invention are those embodiments, wherein the product of shearing rate (1/s) and time (s) is equivalent to any product thereof disclosed herein. In a particular embodiment of the invention the mechanical and chemical lysis methodologies are combined, which enhances the effectivenss of either method.

In a particular practice of the invention the microorganism or microorganism-containing suspension is mixed with a surfactant, most preferably Triton X-100 at concentrations of 0.01-2% w/w d.s. mycelium, more preferably 0.01-1% w/w d.s. mycelium and even more preferably 0.01-0.5% w/w d.s. mycelium. Subsequently, the microorganism/surfactant mixture is preferentially subjected to a shearing process described above, which is for example particularly useful if the microorganism is a fungus, so that its mycelium can be sheared. (Surfactants or detergents are a not particularly limited class of usually organic compounds which typically contain both hydrophobic groups and hydrophilic groups, and thus have amphiphilic character.)

In another preferred practice of the invention the microorganism or the microorganism-containing suspension is mixed with an organic solvent, comprising either amines, ethers or alcohols, such as ethanol at concentrations of 1-30% w/v, more preferably 1-20% w/v and even more preferably 1-5% w/v. Subsequently, the microorganism/surfactant mixture is subjected to a process shearing process described above, which is for example particularly useful if the microorganism is a fungus, so that its mycelium can be sheared.

The specific power input into the slurry can be achieved either by increasing impellor speed of the fermentation vessel, by pumping the fermentation slurry from the root to the target vessel and by high pressure homogenisation in a specific reaction vessel. The addition of a chemical agents, such as salts, organic solvents, enzymes and surfactants significantly reduces the necessary power input to obtain successful mycelia lysis and release of intracellular enzyme components.

In another aspect of the invention the mechanical treatment is accomplished by chemical lysis, which can be accomplished by addition of inorganic salts (osmolysis), enzymes, organic solvents and/or surfactants. In one particular aspect of the invention the mycelia cell lysis is induced by addition of 0.01-10M, more preferably 0.01-5M and even more preferably 0.1-1M inorganic salts such as NaCl, KCl, or other chemical stress agents such as (NH₂)₂CO, CH₆ClN₃.

In another particular aspect of the invention, the chemical lysis reagent may comprise a charged or uncharged surfactant (Biotechnol Lett. (1980) 2, pp.43-48), such as Tween-80 (uncharged), Triton X-100 (uncharged), SDS (negatively charged) or CTAB (positively charged), which are applied at concentrations of 0.1-3%w/w d.s. microbial biomass, more preferably 0.1-1%w/w d.s. mycel and even more preferably at 0.5-1% /w d.s. microbial biomass respectively.

In another aspect of the invention the chemical lysis reagent may comprise an enzyme or enzyme system, such as chitinase (Plant Pathol. (200) 49, pp. 573-589), which is applied at concentrations of 0.01-10%w/w d.s. microbiasl biomass, more preferably 0.01-1%w/w d.s. mycel and even more preferably at 0.01-0.1% /w d.s. mycel respectively followed by an incubation at 20-70°C for 0.1 - 72 h , repectively.

In a particular practice of the invention the mechanical and chemical lysis methodologies are combined, which is believed to enhance the effectiveness of either method.

In a particular practice of the invention the microorganism is mixed with a surfactant, most preferably Triton X-100 at concentrations of 0.01-5% w/w d.s. microorganism biomass, more preferably 0.01-1% w/w d.s. microorganism biomass. Subsequently, the microorganism/surfactant mixture is subjected to volumetric power input of 1 - 500 kW/m³, more preferably 1 - 200 kW/ m³ for 0.1 - 60 min for effective treatment.

In another prefered practice of the invention the microorganism biomass is mixed with an organic solvent, comprising either amines, ethers or alcohols, such as ethanol at concentrations of 5-40% w/v, more preferably 20-40% w/v and even more preferably 30-35% w/v. Subsequently, the microorganism/organic solvent mixture is subjected to volumetric power input of 1 - 500 kW/m³, more preferably 1 - 200 kW/ m³ for 0.1 - 60 min. for effective treatment.

The specific power input into the slurry can be achieved either by increasing impellor speed of the fermentation vessel, by pumping the fermentation slurry from the root to the target vessel and by high pressure homogenisation in a specific reaction vessel. The addition of a chemical agents, such as salts, organic solvents, enzymes and surfanctants significantly reduces the necessary power input to obtain successful mycelia lysis and release of intracellular enzyme components.

It has unexpectedly been found that the release of intracellular enzyme activities from an enzyme producing organism can enhance hydrolysis performance of the applied enzyme system such that lower cellulase and beta-glucosidase dosing regimes can be applied.

Data according to this invention surprisingly show that sheared or otherwise physically or mechanically or chemically processing mycelial biomass prior to hydrolysis will release as much beta-Glucosidase (BGL) as would otherwise have to be extrinsically supplied to obtain the same hydrolysis yield. Shearing and extrinsic addition of BGl activities has an additive effect giving higher sugar yields and enhanced saccharification kinetics.

The release of mycelium-bound enzyme activities also allows for a reduction in cellulase enzyme dosing without compromising hydrolysis kinetics or soluble sugar yields from lignocellulosic biomass. In summary, teachings disclosed herein demonstrate that the utilisation of a pre-treated feedstock as a sole growth medium allows for production of enzyme systems that are very well adapted to decompose this specific lignocellulosic material. Therefore, on site enzyme production as disclosed in this invention allows for maximal flexibility in the choice of lignocellulosic feedstocks and pre-treatment options. Further, the utilisation of the entire spent fermentation slurry for downstream biomass hydrolysis operations allows for a more efficient dosing regime, which enhances product yield and enzyme cost efficiencies.

Enzyme dosing regimes in hydrolysis operations can also be reduced by the release of intracellular enzyme activities from enzyme producing organisms boosting the performance of extracellular enzyme systems.

If a fungus is used for enzyme production, the goal of the processing step d) is rupture of all or parts of the fungus, such as the fungal mycelium.

### [Biomass hydrolysis (step (e)]

To accomplish decomposition of pre-treated lignocellulosic biomass into fermentable, monomeric hexose (i.e. glucose) and pentose (i.e. xylose) sugars, the spent fermentation slurry (supernatant and mycelium) from the integrated enzyme production is added together with pre-treated biomass into a reactor for biomass hydrolysis. As commonly known, hexoses are monosaccharides with six carbon atoms, pentoses are monosaccharides with five carbon atoms.

In a non-limiting example pre-treated biomass slurry having a solids content up to 30 %, preferably 15 - 30% (w/w) may preferably be hydrolysed in a batch mode.

It is obvious to those skilled in the art that the dosing of the hydrolysis enzyme system, incubation temperature, residence time, optional feeding regimes for biomass hydrolysis and hydrolysis efficiency are inherently linked to the particular enzyme system and feedstock applied.

In a particular embodiment of the invention hydrolysis of the pre-treated biomass slurry will be accomplished by enzyme systems produced by the said filamentous fungus *Trichoderma reesei.*

In that instance the pre-treated feedstock slurry is mixed with spent and optionally sheared fermentation slurry containing the hydrolytic enzyme systems. The preferred dosing regime for lignocellulosic feedstocks under these conditions ranges between 0.1-1% w Enzyme/ w feedstock or 1-20 FPU/ g cellulose contained in the feedstock (Zu et al., 2009).

Hydrolysis of pre-treated biomass is accomplished at temperatures between 45 - 55 °C at residence times between 18 - 72 h. In a particularly preferred embodiment of this invention batch hydrolysis of pre-treated cereal straw is accomplished at enzyme dosings of 0,25-0,8% w Enzyme/d.s pretreated feedstock with or without additional beta-glucosidase supplementation and at temperature of 50-53°C within 72h.

Surprisingly even with low enzyme dosing regimes hydrolysis yields above 50% w/w with respect to the total sugars contained in said feedstock could be obtained. These high sugar yields at low enzyme dosing regimes could be achieved due to the integrated production of efficient enzyme systems, as has been described above.

It is optionally comprised in this invention that beta-glucosidase is added in step e) of the process.

Preferably, the hydrolysis reaction is characterized in that the soluble sugars obtained in step e) comprise monomeric C5-and/or C6 sugars, preferably glucose and/or xylose.

In a preferred embodiment of this invention the biomass hydrolysate containing a sugar-rich supernatant and solid lignin rich residues will be subjected to a solid/liquid separation procedure, which may be accomplished by example centrifugation, filtration or simple gravity decanting. It is understood that soluble sugars are all the sugars which can predominantly be found in the liquid phase if a solid/liquid separation, as known to the person skilled in the art, is carried out.

### [Fermentation of biomass hydrolysates]

An optional further aspect of this invention is the fermentation of the biomass hydrolysate obtained in step e). Thus, the invention also comprises the process described above, further characterized in that the sugar-rich phase is further processed. In a particular embodiment, the sugar-rich liquid phase is further processed to ethanol. Microorganisms can be applied within the sense of this invention to convert pentose- and hexose-containing biomass hydrolysates into ethanol.

It is preferred that, in this step, a sugar-rich liquid phase obtainable from step e) is used which comprises a high fermentable sugar content, preferentially being in the order of 15 - 50% w/v. Sugar content in this context is the content of all pentoses and hexoses. In a preferred embodiment of the invention this biomass hydrolysate has a sugar content of 16 - 25% w/v. In a more preferred embodiment of the invention, the biomass hydrolysate also contains additional nutrients (i.e. proteins, salts and sugars) carried over from integrated enzyme production processes.

Fermentation of the biomass hydrolysate to ethanol may be accomplished with microbial strains that show process robustness, high inhibitor / ethanol tolerance, reliable product yields at high and low sugar concentrations. Non-limiting examples of such microorganisms that can convert sugars to ethanol include the bakers yeast *S. cerevisiae, Pichia stipitis, Hansenula polymorpha, Clostridium acetobutylicum, Thermoanaerobacterium saccharolyticum, Zymomonas mobilis, E. coli, Klebsiella oxytoca, and Fusarium oxysporum.*

Selection criteria for process options and choice of organism are process robustness, product/ inhibitor/ temperature tolerance and consistently high ethanol yields (Fischer et al., 2008; Matsushika et al., 2009).

Ethanolic fermentations can be conducted at temperatures between 28 - 70°C for 10 - 48h depending on the temperature optimum and fermentation efficiency of the utilised micro-organism (Matsushika et al., 2009).

Teachings of prior art indicate that theoretical conversion limits for hexose conversion to ethanol can be 51% w/w (i.e. 1 g glucose to 0.51 g ethanol) for yeasts (Matsushika et al., 2009).

In the disclosures described herein it was surprisingly found that the pre-treatment did not influence the performance of biomass hydrolysis or fermentation operations.

Hexose fermentations with yeast strains can be conducted at temperatures of 28 - 35°C, pH 4 - 6 and can be completed in 10 - 48h. As a result of the fermentation a slurry is obtained containing yeast biomass and about 0.5 - 25% w/v ethanol depending on the initial sugar concentration of the biomass hydrolysate and the conversion efficiency for pentose and hexose sugars.

### [Ethanol recovery]

The recovery of ethanol from fermentation liquids can be accomplished by various methods known to those skilled in the art. One such procedure is conventional distillation and rectification process relying on equipment applied in the brewing and chemical industries such as distillation columns. These technologies are well established in the industry and alternative apparatus are known per se (Leland, 2008, Cardona and Sanchez, 2007). In an alternative embodiment of the invention ethanol is recovered by a stripping technology, non-limiting examples of such technology being vacuum stripping, gas stripping, spray evaporation.

The practices of the current invention can result in ethanol product of 90-100% v/v. This product can optionally be mixed with additives or alternatively processed to formulate a product that will be shipped to the end customer.

### Definition of Terms:

In the present invention a number of terms are used, which are defined below.

The terms "Biomass" and "lignocellulosic biomass" refer to any cellulosic or lignocellulosic material and includes materials comprising cellulose, and optionally further comprising hemicellulose, lignin, starch, polysaccharides, oligosaccharides and/or monosaccharides. Biomass may also comprise additional components, such as protein, lipids, waxes, phenolics and steroids. Biomass may generally be derived from a single source, or comprises a mixture derived from more than one source; for example, biomass could comprise a mixture of wheat spelts and weath straw, or a mixture of grass and leaves. Non limiting examples of biomass are bioenergy crops, agricultural residues, municipal solid waste, industrial solid waste, sludge from paper manufacture, yard waste, wood and forestry waste. Specifically non-limimting examples of pure biomass include oat spelts, corn cobs, crop residues such as corn husks, corn stover, grasses, wheat, wheat straw, barley, barley straw, hay, rice straw, switchgrass, waste paper, sugar cane bagasse, sorghum, soy, components obtained from milling of grains, trees, branches, roots, leaves, wood chips, sawdust, shrubs and bushes, vegetables, fruits, flowers and animal manure.

The term "mechanical de-sizing" refers to any mechanical method for reducing the particle size of biomass. Non-limiting examples of de-sizing applications are hammer milling or crushing.

The term "pre-treated biomass" means biomass that has been subjected to a physicochemical treatment prior to saccharification. Treatments such as pre-treatments are further described herein.

The term "lignocellulosic" refers to a composition comprising both lignin and cellulose. Lignocellulosic material may also comprise hemicellulose.

The term "cellulosic" refers to a composition comprising cellulose.

The term "saccharification" or "biomass hydrolysis" refers to the production of fermentable sugars from polysaccharides.

The term "fermentable sugar" refers to oligosaccharides and monosaccharides that can be used as a carbon source by a microorganism in a fermentation process to make products such as ethanol.

The term "hydrolysate" refers to the product of saccharification, which contains the sugars produced in the saccharification process, the remaining non-hydrolyzed biomass, and the enzymes used for biomass hydrolysis

The term "slurry" refers to a mixture of insoluble material and a liquid.

The term "mixable slurry" refers to a slurry that becomes substantially homogeneous under the action of the agitation system to which it is subjected. "Mixability" refers to this property of a slurry.

The term "thoroughly mixed slurry" refers to a state where the components of the slurry are substantially evenly distributed (homogeneous) throughout the slurry.

By "dry weight" or "d.w." of biomass is meant the weight of the biomass having all or essentially all water removed. Dry weight is typically measured according to American Society for Testing and Materials (ASTM) Standard E1756-01 (Standard Test Method for Determination of Total Solids in Biomass) or Technical Association of the Pulp and Paper Industry, Inc. (TAPPI) Standard T-412 om-02 (Moisture in Pulp, Paper and Paperboard).

The term "dry weight" (d.w.) or "dry substance" (d.s.) refers to the total amount of biomass dry weight added into a batch or fed batch system reactor, calculated at the time of addition, as a percent of the total weight of the reacting composition in the reactor at the end of the run.

The term "saccharification enzymes" or "hydrolysing enzymes" may refer to a saccharification enzyme system comprised of one or more enzymes (wherein more than one is preferred), used to hydrolyze the polymeric biomass thereby releasing oligosaccharides and/or monosaccharides into a hydrolysate. A saccharification enzyme system comprises one or more enzymes selected from the group "glycosidases" which hydrolyze the ether linkages of di-, oligo-, and polysaccharides and are found in the enzyme classification EC 3.2.1.x of the general group "hydrolases" (EC 3.). Additionally, further enzymes may be present which may or may not belong to this group. Glycosidases useful in the present method can be categorized by the biomass component that they hydrolyze. Glycosidases useful for the present method include cellulose-hydrolyzing glycosidases (for example, cellulases, endoglucanases, exoglucanases, cellobiohydrolases, beta-glucosidases), hemicellulose-hydrolyzing glycosidases, called hemicellulases, (for example, xylanases, endoxylanases, exoxylanases, beta-xylosidases, arabinoxylanases, mannases, galactases, pectinases, glucuronidases), and starch-hydrolyzing glycosidases (for example, amylases, α-amylases, β-amylases, glucoamylases, α-glucosidases, isoamylases). In addition, it may be useful to add other activities to the saccharification enzyme system such as peptidases (EC 3.4.x.y), lipases (EC 3.1.1.x and 3.1.4.x), ligninases (EC 1.11.1.x), and feruloyl esterases (EC 3.1.1.73) to help release polysaccharides from other components of the biomass. It is well known in the art that microorganisms that produce polysaccharide-hydrolyzing enzymes often exhibit an activity, such as cellulose degradation, that is catalyzed by several enzymes or a group of enzymes having different substrate specificities. Thus, a "cellulase" from a microorganism may comprise a group of enzymes, all or some of which may contribute to the cellulose-degrading activity. Commercial or non-commercial enzyme preparations, such as cellulase, may comprise numerous enzymes depending on the purification scheme utilized to obtain the enzyme. Thus, the saccharification enzymes used in the present method comprise at least one "cellulase", and this activity may be catalyzed by more than one enzyme. Optionally, the saccharification enzymes used in the present method may comprise at least one hemicellulase, generally depending on the type of pre-treated biomass used in the present process. For example, hemicellulase is typically not needed when saccharifying biomass pre-treated with acid and is typically included when saccharifying biomass pre-treated under neutral or basic conditions. Saccharification enzymes may be obtained commercially, such as Spezyme® CP cellulase (Genencor International, Rochester, N.Y.) and Multifect® xylanase (Genencor). In addition, saccharification enzymes may be produced biologically, including using recombinant microorganisms. New saccharification enzymes may be developed, which may be used in the present process.

SSF stands for simultaneous saccharification and fermentation.

### Examples

The following terms are used:
"HPLC" is High Performance Liquid Chromatography, "C" is Centigrade, "kPa" is kiloPascal, "m" is meter, "mm" is millimeter, "kW" is kilowatt, "µm" is micrometer, "µL" is microliter, "mL" is milliliter, "L" is liter, "min" is minute, "mM" is millimolar, "cm" is centimeter, "g" is gram, "kg" is kilogram, "wt" is weight, "hr" is hour, "temp" or "T" is temperature, "theoret" is theoretical, "pretreat" is pre-treatment, "DWB" is dry weight of biomass , "ASME" is the American Society of Mechanical Engineers, "s.s." is stainless steel, in" is inch, "PSD" is particle size distribution, "d-50" is the particle diameter where 50% of the cumulative volume of the particles is below this size, "d-95" refers to a particle diameter where 95% of the cumulative volume of the particles is below this size, "rpm" is revolutions per minute.

Sulfuric acid, ammonium hydroxide, acetic acid, acetamide, yeast extract, glucose, xylose, sorbitol, MgSO 4 · 7H 2 O, phosphoric acid and citric acid were obtained from Sigma-Aldrich (St. Louis, Mo.).

The composition of biomass is measured by any one of the standard methods well known in the art, such as ASTM E1758-01 "Standard method for the determination of carbohydrates by HPLC".

### Example 1: Measurement of Monomeric Sugars

To determine the progress of straw hydrolysis, samples are taken at appropriate time intervals and the soluble sugar content is determined by HPLC.

Soluble sugars (such as glucose, cellobiose, xylose, xylobiose, galactose, arabinose, and mannose) in saccharification liquor were measured by HPLC (Agilent Technologies, Palo Alto, Calif.).

Monosaccharides were directly measured in the hydrolysate. The insoluble matter was removed from the hydrolysate by centrifuge. The pH of the separated liquid was adjusted, if necessary, with sulfuric acid. The separated liquid was diluted, if necessary, then filtered by passing through a 0.2 µm syringe filter directly into an HPLC vial.

For analysis of total dissolved sugars, 10 ml of diluted sample was placed in a pressure vial and 349 µl of 75% H2SO4 was added. The vial was capped and placed in the Autoclave for an hour to hydrolyze all sugars to monosaccharides. The samples were cooled and their pH was adjusted by sodium carbonate to the necessary pH, then the samples were filtered into the HPLC vials and analyzed by HPLC. After the run, concentrations in the sample were determined from standard curves for each of the compounds.

### Example 2: Physicochemical pre-treatment of wheat straw

Wheat straw was milled to a particle size of less than 2 cm. Subsequently, the milled straw was mixed with water and H₂SO₄ was added as a pre-treatment catalyst followed by hydrothermal treatment under high pressure.

The resulting suspension of pre-treated feedstock was then used for production of hydrolysis enzyme and for the saccharification process in downstream operations.

### Example 3: Production of hydrolysis enzymes using pre-treated feedstock

Hydrolysis enzymes (i.e. cellulases and hemicellulases) for the conversion of lignocellulosic material into component sugars were produced in submergred cultures of the filamentous fungus Trichoderma reesei.

In a primary culturing step, seed cultures were grown in 21 shake flask filled with 400ml culture broth that was supplemented with 2 % w/v pre-treated biomass (see example 2) and 0.5 % v/v corn steep liquor. The medium was inoculated with a preparation of fungal spores having an Optical Density (OD) at 600nm of 10. The shake flask cultures were grown for 48h at 30°C and pH 5 under constant agitation of 250 rpm.

The seed culture scale-up was conducted in bioreactors with a working volume of 51. The culture medium composition was identical as in the primary seed culture set-up. Each bioreactor was inoculated with 10% v/v of the primary seed culture. The culture was grown at 30°C (pH 5) for 48h with a constant agitation of 350rpm.

The main enzyme production was carried out in a 1501 bioreactor with a working volume of 1001. Culture media was identical to the previous seed cultures. However, for production of hydrolysis enzymes 8% w/v pre-treated biomass and 2% v/v corn steep liquor was added. The culture broth was inoculated with 5% v/v of the secondary seed culture. The enzyme-producing fungus Trichoderma reesei was then grown at 30°C under a constant agitation of 350rpm. Growth commenced for 5 days at a constant pO2 of 25% to ensure optimal enzyme production under aerobic conditions. After the 5 day culturing conditions the whole enzyme containing fermentation slurry containing residues of pre-treated biomass, spent fermentation broth (medium) and fungal mycelium was harvested.

The total soluble enzyme (protein) concentration in the fermentation broth (supernatant) was determined by the Bradford method with bovine serum albumin as a reference standard (Bradford, M., 1976).

In downstream hydrolysis experiments either the enzyme-containing supernatant or the whole fermentation slurry (supernatant + pre-treated biomass + fungal mycelium) was utilised.

### Example 4: Straw Hydrolysis

Pre-treated wheat straw was hydrolysed either with cellulase systems containing (i) the supernatant described in example 3, (ii) the complete fungal suspension (fungal mycelium + biomass + supernatant) or (iii) the complete fungal suspension whereby the fungal mycelium had been sheared prior to straw hydrolysis. Hydrolysis experiments were also optionally conducted in presence of externally added beta-glucosidase (BGL).

Hydrolysis reactions were conducted in a 11 reaction volume with 20 % w/w d.s. pre-treated straw at 50°C for 72h in a medium that was adjusted to pH 5. The hydrolysis enzyme dosing regime was 0.5% w Cellulase/ w pre-treated straw. The enzyme dosing was referenced to the total protein concentration in the spent fermentation broth as determined in example 2. Optionally reactions were conducted with additional BGL activity (Novo 188, Novozymes, Denkmark). The BGL activity was dosed at 2 Cellobiose Units (CBU)/mg cellulase. CBUs were determined as described by Prior et al. (2008).

The saccharification reactions were carried out in a bioreactor system under constant agitation (50 rpm) to ensure equal reactions conditions. In particular experiments the hydrolysis enzyme containing fermentation slurry was sheared by increasing impellor (L5M, Silverson Machines Ltd.) speed to 8000 rpm for 30 min prior to downstream lignocellulose hydrolysis process. To determine the hydrolysis kinetics and glucose yields, samples were taken at 3, 7, 24 and 48 h. The apparent glucose release was measured by HPLC methodology as described above. Results of the different hydrolysis set-ups are shown in Fig. 2.

Data in Fig. 2 indicate that shearing of fungal biomass prior to hydrolysis enhances hydrolysis kinetics and terminal glucose yields, if the enzyme-containing *Trichoderma* fungal suspension (see example 3) is used for hydrolysis of pre-treated straw in the absence of BGL supplementation, d.

Pre-treated straw hydrolysis experiments conducted only with BGL supplemented fermentation broth (supernatant) showed similar hydrolysis kinetics and terminal glucose yields as reactions conducted with whole sheared fermentation slurry in the absence of BGL.

The hydrolysis reaction with sheared whole fermentation slurry (i.e. fungal mycelium, biomass and supernatant) that was additionally supplemented with BGL showed the most favourable hydrolysis kinetics and sugar yield compared to all other hydrolysis processes examined. This surprising effect may be due to the release of enzyme activities intracellular and/or surface-bound to the microorganism, that act synergistically with soluble cellulase systems in the fermentation broth.

### Description of Figures:

Fig. 1: Biomass conversion to ethanol conversion process including mycelial carryover from on site enzyme production. L= lignin, P= pentose, H= hexose. Fig. 1 is a block diagram generally illustrating the biorefining process used to convert lignocellulose into ethanol via a fermentation pathway in accordance with the present invention.
Fig. 2: Glucose release from pre-treated straw. Hydrolysis conditions: 20% w/w d.s. pre-treated biomass, Cellulase enzyme dosing: 0, 5% w soluble Enzyme/w pre-treated biomass, Optional BGL dosing: 2 CBU/mg cellulase enzyme, Sampling at 3, 7, 24, 48, 72 h. Biomass hydrolysis was conducted either with enzyme containing fermentation broth or sheared/unsheared whole fermentation slurry (broth supernatant + biomass + fungal mycelium) in the absence and presence of additional BGL activity.

### Literature:

Chauve et al. (2010) Biotechnology for Biofuels 2010, 3:3
Bradford, M. (1976), Anal. Biochem. 72:248-254
Prior B.A., Day D.F. (2008) Appl. Biochem. Biotechnol. 146(1-3), pp. 151-64
Pejo, E.T., Oliva, J.M. and Ballestros, M. (2008) Realistic approach for full-scale bioethanol production from lignocellulose: a review J. Sci& Indust. Research 67, pp. 874-884
Howard, R.L., Abotsi, E., van Rensburg, J. Howard, S. (2003) Lignocellulose biotechnology: issues of bioconversion and enzyme production. Afri. J. Biotechnol. 2, pp. 602-619
Sluiter, B. Hames, R. Ruiz, C. Scarlata, J. Sluiter, D. Templeton, and D. Crocker (2008) Determination of Structural Carbohydrates and Lignin in Biomass. Laboratory Analytical Procedure (LAP) Technical Report NREL/TP-510-42618, http://www.nrel.gov/biomass/pdfs/42618.pdf
Kumar R, Mago G, Balan V, Wyman CE. (2009a) Physical and chemical characterizations of corn stover and poplar solids resulting from leading pretreatment technologies. Bioresour. Technol. 100(17), pp.3948-62.
Kumar R, Wyman CE. (2009b) Effects of cellulase and xylanase enzymes on the deconstruction of solids from pretreatment of poplar by leading technologies. Biotechnol Prog. 2009, 25(2), pp. 302-14.
Kumar R, Wyman CE. (2009c) Effect of additives on the digestibility of corn stover solids following pretreatment by leading technologies. Biotechnol Bioeng. 2009, Apr 15; 102(6):1544-57.
Wyman CE, Dale BE, Elander RT, Holtzapple M, Ladisch MR, Lee YY. (2005) Comparative sugar recovery data from laboratory scale application of leading pretreatment technologies to corn stover. Bioresour. Technol. 96(18), pp. 2026-32.
Chandra, R.R., Bura, R., Mabee, W.E., Berlin, A., Pan, X:, Saddler, J.N. (2007) Substrate Pretreatment: The key to effective enzymatic hydrolysis of lignocellulosics? Adv. Biochem. Engin/Biotechnol. 108, pp. 67-93.
Margeot, A., Han-Hägerdahl, B., Edlund, M., Slade, R., Monot, F. (2009) New improvements for lignocellulosic ethanol. Curr. Opin.Biotechnol. 20, pp. 1-9.
Chandrakant, P., Bisaria, V. S. (1998) Simultaneous bioconversion of cellulose and hemicellulose to ethnaol. Crit. Rev. Biotechnol. 18, pp. 295-331
Xiao, Z., Zhang, X., Gregg, D.J., Saddler, J.N. (2004a) Effects of sugar inhibition on cellulose and beta-glucosidase during enzymatic hydrolysis of softwood substrates. Appl. Biochem. Biotechnol. 113-116, pp. 1115-1125
Hodge, D.B., Karim, M.N., Schell, D.J., McMillan, J.D. (2009) Model-based fed-batch for high-solids enzymatic cellulose hydrolysis. Appl Biochem Biotechnol. 152, pp. 88-107
Reith, J.H., den Uli, H., van Veen, H., de Laat, W.T.A.M., Niessen, J.J., de Jong, E., Elbersen, H.W., Weusthuis, R., van Dijken, J.P., Raamsdonk, L. (2002) Co-prodcution of bioethanol, electrcity, and heat from biomass residues. In: Twelfth European Conference and Technology Exhibition on Biomass for Energy, Industry and Climate Protection, Amsterdam, The Netherlands.
Palmquist, E., Hahn-Hägerdahl (2000a) Fermentation of lignocellulosic hydrolysates. I: inhibition and detoxification Bioresource Technology 74, pp 17-24
Palmquist, E., Hahn-Hägerdahl (2000b) Fermentation of lignocellulosic hydrolysates. II: inhibitors and mechanisms of inhibition. Bioresource Technol. 74, pp. 25-33
Jing, X., Zhang, X. and Bao, J. (2009) Inhibition Performance of Lignocellulose Degradation Products on Industrial Cellulase Enzymes During Cellulose Hydrolysis Applied Biochemistry and Biotechnology 159, pp. 696-707
Xiao, Z., Storms, R. and Tsang, A. (2004b) Microplate-based filter paper assay to measure total cellulase activity. Biotechnology and Bioengineering 88:832-837
Bobey, D., Ederer, G.M. (1981) Rapid detection of yeast enzymes by using 4-methylumbelliferyl substrates J.Clin.Microbiol.,pp. 2, 393-394
Maki, M., Leung, K.T., Qin, W. (2009) The prospects of cellulose producing bacteria for the bioconversion of lignocellulosic biomass. Int. J. Biol. Sci. 5, pp. 500-516
Lynd, L.R., Weimer, P.J., van Zyl, W.H., Pretorius, I.S. (2002) Microbial cellulose utilisation: Fundamentals and biotechnology. Microbiol. and Molecular. Biology Rev. 66, pp. 506-577
Shewale JG. Beta-Glucosidase: its role in cellulase synthesis and hydrolysis of cellulose. (1982) Int J Biochem. 14, pp. 435-43
Seidle, H.F., Marten, I., Shoseyov, O., Huber, R.E. (2004), Physical and kinetic properties of the family 3 beta-glucosidase from Aspergillus niger which is important for cellulose breakdown. The Protein J. 23, pp. 11-23
Szijarto, N., Szengyel, Z., Liden, G., Reczey, K. (2004) Dynamics of cellulase prodcution by glucose grown cultures of trichoderma reesei RUT-C30 as a response to addition of cellulose Appl. Biochem. Biophys. 115, pp. 115-124
Kristensen, J.B., Felby, C., Jorgensen, H. (2009) Yield-determining factors in high-solids enzymatic hydrolysis of lignocellulose Biotech. for Biofuels 2, pp. 1-22
Fischer, C.R., Klein-Marcuschamer, D., Stephanopoulos, G. (2008) Selection and optimization of microbial hosts for biofuels production. Metab. Eng. 10(6), pp. 295-304.
Shaw A.J., Podkaminer K.K., Desai S.G., Bardsley J.S., Rogers S.R., Thorne P.G., Hogsett D.A., Lynd L.R. (2008) Metabolic engineering of a thermophilic bacterium to produce ethanol at high yield. Proc. Natl. Acad. Sci. 105(37), pp. 13769-74
Campell, J.A., Hansen, R.W., Wilson, J.R. (1999) Cost effective colorimetric microtitre plate enzymic assays for sucrose, glucose and fructose in sugarcane tissue extracts. J. Sci. Food. Agicult. 79, pp. 232-236
Matsushika A, Inoue H, Murakami K, Takimura O, Sawayama S. (2009) Bioethanol production performance of five recombinant strains of laboratory and industrial xylose-fermenting Saccharomyces cerevisiae. Bioresour Technol. 2009 Apr; 100(8) :2392-8.
Nigam JN. (2001) Ethanol production from hardwood spent sulfite liquor using an adapted strain of Pichia stipitis. J Ind Microbiol Biotechnol. 26(3), pp. 145-50.
Laplace JM, Delgenes JP, Moletta R, Navarro JM. (1992) Alcoholic glucose and xylose fermentations by the coculture process: compatibility and typing of associated strains. Can J Microbiol. 38(7), pp. 654-8
Tolan, J.S. (2002): Iogens process for producing ethanol from cellulosic biomass. Clean Technol. Environ. Policy. 3, pp. 339-345
Cardona, C.A., Sanchez, O.J. (2007) Fuel ethanol production: Process design trends and integration opportunities Bioresource Technol. 98, pp. 2415-2457
Jorgensen, H., Vibe-Pedersen, J.,Larsen, J., Felby C. (2006) Liquefaction of lignocellulose at high-solids concentrations. Biotechnology and Bioengineering 96, pp. 862 - 870
Chadha B.S., Kanwar S.S., Saini H.S., Garcha H.S. (1995) Hybrid process for ethanol production from rice straw. Acta Microbiol Immunol Hung. 42(1):53-9.
Hennessey, S.M. Seapan, M., Elander, R.T., Tucker, M.P. (2006) Process for concentrated biomass saccharification WO2009/045651A2
Leland, M.V. (2008) Separation technologies for recovery and dehydration of alcohols from fermentation broth. Biofuel, Biorpod.Bioref. 2, pp. 553-588
Rao, M., Desphpande, V., Seeta, R., Srinivasan, M.C., Mishra, C. (1985) Hydrolysis of sugarcane Bagasse by mycelium biomass from penicillium funiculosum. Biotechnol. Bioengineer 27, 00. 1070-72

## Claims

1. A process for degrading pre-treated lignocellulosic biomass comprising the steps:
c) cultivating a microorganism capable of producing at least one enzyme having cellulolytic and/or hemicellulolytic activity in a growth medium, thereby obtaining a microorganism-rich suspension comprising said at least one enzyme;
d)processing the microorganism and/or the micro-organism-rich suspension of step c by
(i) a mechanical treatment comprising subjection to a volumetric power input of 1-500 kW/m3, for a duration of 0.1-60 min;
and/or
(ii) a mechanical treatment which is selected from a treatment with a mixer, a treatment with an homogenizer, and a treatment with a mill; ,
and/or
(iii) a mechanical treatment which involves pumping of the microorganism-containing fermentation slurry from the fermentation vessel to the hydrolysis vessel;
e) subjecting pre-treated lignocellulosic biomass together with the product of step d) to a reactor for biomass hydrolysis to obtain soluble sugars.

2. The process according to claim 1, wherein the pre- treated lignocellulosic biomass has been obtained from lignocellulosic biomass by a physico-chemical treatment.

3. The process according to any one of the preceding claims, wherein the growth medium of step c) comprises lignocellulosic biomass, which has preferably been pre-treated.

4. A process for degrading lignocellulosic biomass comprising the steps:
a)physico-chemical pre-treatment of lignocellulosic biomass to obtain a pre-treated slurry;
b) separating the pre-treated slurry of step a) into two parts, part A and part B;
c) incorporating part A into a raw growth medium to yield a final growth medium, and cultivating at least one microorganism capable of producing at least one enzyme having cellulolytic and/or hemicellulolytic activity in the final growth medium, thereby obtaining a microorganism-rich suspension comprising said at least one enzyme;
d) processing the microorganism and/or the micro-organism-rich suspension of step c, wherein said processing comprises a mechanical treatment which is one or more of the following:
(i) a mechanical treatment comprising subjection to a volumetric power input of 1-500 kW/m3 for a duration of 0.1-60 min;
and/or
(ii) a mechanical treatment which is selected from a treatment with a mixer, a treatment with an homogenizer, and a treatment with a mill;
and/or
(iii)a mechanical treatment which involves pumping of the microorganism-containing fermentation slurry from the fermentation vessel to the hydrolysis vessel;
e) subjecting together part B and the product of step d) to a reactor for biomass hydrolysis to obtain soluble sugars.

5. The process according to claim 4, wherein the mechanical treatment in step d) comprises subjection to a volumetric power input of 1-500 kW/m³ for a duration of 1-30 min.

6. The process according to claim 4 or 5, wherein the mechanical treatment in step d) is selected from a treatment with a mixer, a treatment with an homogenizer, and a treatment with a mill, wherein the mechanical treatment can add mechanical shearing stress or grinding force to the microorganism and can disrupt or destroy the cell membranes and/or the cell wall structures.

7. The process according to any one of claims 4 to 6, wherein the mechanical treatment in step d) involves pumping of the microorganism-containing fermentation slurry from the fermentation vessel to the hydrolysis vessel.

8. The process according to any one of the preceding claims, wherein, when step d) involves pumping, the shearing rate, to which the microorganism-containing slurry is subjected, is in the range of 1600 - 50000 1/s.

9. The process according to claim 8, wherein the treatment is done for a period of 0.01 to 100 s.

10. The process according to any one of the preceding claims, wherein treatment comprises a treatment with ultrasonic.

11. The process according to any one of the preceding claims, wherein step d) comprises a chemical treatment, which is a treatment with one or more chemical agents, selected from the group consisting of salts, organic solvents, surfactants and enzymes.

12. The process according to any one of claims 1 to 4, wherein step d) comprises a mechanical treatment according to claim 5 to 9 and a treatment with one or more chemical agents according to claim 11.

13. The process according to any one of claims 4 to 12, wherein part A is the minor part of the pre-treated slurry obtained in step a, 1 to 20 % (weight dry solids).

14. The process according to any one of the preceding claims, wherein the microorganism is a fungus, which is selected from the group consisting of: *Trichoderma sp.,* Aspergillus sp., Penicillium sp. and *Talaromyces sp..*

15. The process according to claim 14, wherein the microorganism is *Trichoderma reesei.*

## Patentansprüche

1. Verfahren zum Abbauen von vorbehandelter Lignocellulose-Biomasse, umfassend die Schritte:
c) Kultivieren eines Mikroorganismus, der fähig ist, wenigstens ein Enzym mit cellulolytischer und/oder hemicellulolytischer Aktivität herzustellen, in einem Wachstumsmedium, um so eine Mikroorganismus-reiche Suspension zu erhalten, die das wenigstens eine Enzym umfasst;
d) Verarbeiten des Mikroorganismus und/oder der Mikroorganismus-reichen Suspension aus Schritt c) durch
(i) eine mechanische Behandlung, umfassend Unterwerfen an eine volumetische Leistungszufuhr von 1-500 kW/m³ für eine Dauer von 0,1-60 min;
und/oder
(ii) eine mechanische Behandlung, die ausgewählt ist aus einer Behandlung mit einem Mischer, einer Behandlung mit einem Homogenisator und einer Behandlung mit einer Mühle;
und/oder
(iii) eine mechanische Behandlung, die Pumpen der Mikroorganismus-enthaltenden Fermentationsaufschlämmung aus dem Fermentationsgefäß zu dem Hydrolysegefäß umfasst;
e) Unterwerfen der vorbehandelten Lignocellulose-Biomasse zusammen mit dem Produkt aus Schritt d) an einen Reaktor für die Biomasse-Hydrolyse, um lösliche Zucker zu erhalten.

2. Verfahren gemäß Anspruch 1, wobei die vorbehandelte Lignocellulose-Biomasse durch eine physikalisch-chemische Behandlung aus Lignocellulose-Biomasse erhalten worden ist.

3. Verfahren gemäß einem der vorstehenden Ansprüche, wobei das Wachstumsmedium bei Schritt c) Lignocellulose-Biomasse umfasst, die vorzugsweise vorbehandelt worden ist.

4. Verfahren zum Abbauen von Lignocellulose-Biomasse, umfassend die Schritte:
a) physikalisch-chemische Vorbehandlung von Lignocellulose-Biomasse, um eine vorbehandelte Aufschlämmung zu erhalten;
b) Auftrennen der vorbehandelten Aufschlämmung aus Schritt a) in zwei Teile, Teil A und Teil B;
c) Einverleiben von Teil A in ein rohes Wachstumsmedium, um ein fertiges Wachstumsmedium zu erhalten, und Kultivieren wenigstens eines Mikroorganismus, der fähig ist, wenigstens ein Enzym mit cellulolytischer und/oder hemicellulolytischer Aktivität herzustellen, in dem fertigen Wachstumsmedium, um so eine Mikroorganismus-reiche Suspension zu erhalten, die das wenigstens eine Enzym umfasst;
d) Verarbeiten des Mikroorganismus und/oder der Mikroorganismus-reichen Suspension aus Schritt c), wobei die Verarbeitung eine mechanische Behandlung umfasst, die eine oder mehrere der folgenden ist:
(i) eine mechanische Behandlung, umfassend Unterwerfen an eine volumetische Leistungszufuhr von 1-500 kW/m³ für eine Dauer von 0,1-60 min;
und/oder
(ii) eine mechanische Behandlung, die ausgewählt ist aus einer Behandlung mit einem Mischer, einer Behandlung mit einem Homogenisator und einer Behandlung mit einer Mühle;
und/oder
(iii) eine mechanische Behandlung, die Pumpen der Mikroorganismus-enthaltenden Fermentationsaufschlämmung aus dem Fermentationsgefäß zu dem Hydrolysegefäß umfasst;
e) Unterwerfen des Produkts aus Schritt d) zusammen mit Teil B an einen Reaktor für die Biomasse-Hydrolyse, um lösliche Zucker zu erhalten.

5. Verfahren gemäß Anspruch 4, wobei die mechanische Behandlung bei Schritt d) Unterwerfen an eine volumetische Leistungszufuhr von 1-500 kW/m³ für eine Dauer von 1-30 min umfasst.

6. Verfahren gemäß Anspruch 4 oder 5, wobei die mechanische Behandlung bei Schritt d) ausgewählt ist aus einer Behandlung mit einem Mischer, einer Behandlung mit einem Homogenisator und einer Behandlung mit einer Mühle, wobei die mechanische Behandlung mechanische Scherspannung oder Mahlkraft an die Mikroorganismen aufbringen kann und die Zellmembranen und/oder die Zellwandstrukturen aufbrechen oder zerstören kann.

7. Verfahren gemäß einem der Ansprüche 4 bis 6, wobei die mechanische Behandlung bei Schritt d) Pumpen der Mikroorganismus-enthaltenden Fermentationsaufschlämmung aus dem Fermentationsgefäß zu dem Hydrolysegefäß umfasst.

8. Verfahren gemäß einem der vorstehenden Ansprüche, wobei, wenn Schritt d) Pumpen umfasst, die Scherrate, der die Mikroorganismus-enthaltende Aufschlämmung unterworfen wird, in dem Bereich von 1600-50000 l/s liegt.

9. Verfahren gemäß Anspruch 8, wobei die Behandlung über einen Zeitraum von 0,01 bis 100 s durchgeführt wird.

10. Verfahren gemäß einem der vorstehenden Ansprüche, wobei die Behandlung eine Behandlung mit Ultraschall umfasst.

11. Verfahren gemäß einem der vorstehenden Ansprüche, wobei Schritt d) eine chemische Behandlung umfasst, die eine Behandlung mit einem oder mehreren chemischen Mitteln ausgewählt aus der Gruppe bestehend aus Salzen, organischen Lösungsmitteln, grenzflächenaktiven Mitteln und Enzymen ist.

12. Verfahren gemäß einem der Ansprüche 1 bis 4, wobei Schritt d) eine mechanische Behandlung gemäß Anspruch 5 bis 9 und eine Behandlung mit einem oder mehreren chemischen Mitteln gemäß Anspruch 11 umfasst.

13. Verfahren gemäß einem der Ansprüche 4 bis 12, wobei Teil A der kleinere Teil der bei Schritt a) erhaltenen vorbehandelten Aufschlämmung ist, 1 bis 20 % (Trockensubstanzgewicht).

14. Verfahren gemäß einem der vorstehenden Ansprüche, wobei der Mikroorganismus ein Pilz ist, der ausgewählt ist aus der Gruppe bestehend aus: *Trichoderma sp., Aspergillus sp., Penicillum sp.* und *Talaromyces sp..*

15. Verfahren gemäß Anspruch 14, wobei der Mikroorganismus *Trichoderma reesei* ist.

## Revendications

1. Procédé de dégradation de biomasse lignocellulosique prétraitée comprenant les étapes de :
c) culture d'un micro-organisme capable de produire au moins une enzyme ayant une activité cellulolytique et/ou hémicellulolytique dans un milieu de culture, de manière à obtenir une suspension riche en micro-organisme comprenant ladite au moins une enzyme ;
d) traitement du micro-organisme et/ou de la suspension riche en micro-organisme de l'étape c par
(i) un traitement mécanique comprenant la soumission à une application de puissance volumétrique de 1 à 500 kW/m³, pendant une durée de 0,1 à 60 min ;
et/ou
(ii) un traitement mécanique qui est choisi parmi un traitement avec un mélangeur, un traitement avec un homogénéisateur, et un traitement avec un broyeur ;
et/ou
(iii) un traitement mécanique qui met en oeuvre le pompage de la suspension concentrée de fermentation contenant le micro-organisme depuis la cuve de fermentation vers la cuve d'hydrolyse ;
e) soumission de la biomasse lignocellulosique prétraitée conjointement avec le produit de l'étape d) à un réacteur pour hydrolyse de la biomasse pour obtenir des glucides solubles.

2. Procédé selon la revendication 1, dans lequel la biomasse lignocellulosique prétraitée a été obtenue à partir de biomasse lignocellulosique par un traitement physico-chimique.

3. Procédé selon l'une quelconque des revendications précédentes, dans lequel le milieu de culture de l'étape c) comprend une biomasse lignocellulosique, qui a de préférence été prétraitée.

4. Procédé de dégradation de biomasse lignocellulosique comprenant les étapes de :
a) prétraitement physico-chimique de biomasse lignocellulosique pour obtenir une suspension concentrée prétraitée ;
b) séparation de la suspension concentrée prétraitée de l'étape a) en deux parties, partie A et partie B ;
c) incorporation de la partie A dans un milieu de culture brut pour obtenir un milieu de culture final, et cultures d'au moins un micro-organisme capable de produire au moins une enzyme ayant une activité cellulolytique et/ou hémicellulolytique dans le milieu de culture final, de manière à obtenir une suspension riche en micro-organisme comprenant ladite au moins une enzyme ;
d) traitement du micro-organisme et/ou de la suspension riche en micro-organisme de l'étape c, ledit traitement comprenant un traitement mécanique qui est un ou plusieurs des suivants :
(i) un traitement mécanique comprenant la soumission à une application de puissance volumétrique de 1 à 500 kW/m³, pendant une durée de 0,1 à 60 min ;
et/ou
(ii) un traitement mécanique qui est choisi parmi un traitement avec un mélangeur, un traitement avec un homogénéisateur, et un traitement avec un broyeur ;
et/ou
(iii) un traitement mécanique qui met en oeuvre le pompage de la suspension concentrée de fermentation contenant le micro-organisme depuis la cuve de fermentation vers la cuve d'hydrolyse ;
e) soumission conjointement de la partie B et du produit de l'étape d) à un réacteur pour hydrolyse de la biomasse pour obtenir des glucides solubles.

5. Procédé selon la revendication 4, dans lequel le traitement mécanique dans l'étape d) comprend la soumission à une application de puissance volumétrique de 1 à 500 kW/m³, pendant une durée de 1 à 30 min.

6. Procédé selon la revendication 4 ou 5, dans lequel le traitement mécanique dans l'étape d) est choisi parmi un traitement avec un mélangeur, un traitement avec un homogénéisateur, et un traitement avec un broyeur, le traitement mécanique pouvant ajouter une contrainte de cisaillement mécanique ou une force de broyage au micro-organisme et peut rompre ou détruire les membranes cellulaires et/ou les structures de parois cellulaires.

7. Procédé selon l'une quelconque des revendications 4 à 6, dans lequel le traitement mécanique dans l'étape d) met en oeuvre le pompage de la suspension concentrée de fermentation contenant le micro-organisme depuis la cuve de fermentation vers la cuve d'hydrolyse.

8. Procédé selon l'une quelconque des revendications précédentes, dans lequel, lorsque l'étape d) met en oeuvre le pompage, le taux de cisaillement, auquel la suspension concentrée contenant le micro-organisme est soumis, est dans la plage de 1600 à 50000 1/s.

9. Procédé selon la revendication 8, dans lequel le traitement est effectué pendant une durée de 0,01 à 100 s.

10. Procédé selon l'une quelconque des revendications précédentes, dans lequel le traitement comprend un traitement avec des ultrasons.

11. Procédé selon l'une quelconque des revendications précédentes, dans lequel l'étape d) comprend un traitement chimique, qui est un traitement avec un ou plusieurs agents chimiques, choisis dans le groupe constitué de sels, de solvants organiques, de tensioactifs et d'enzymes.

12. Procédé selon l'une quelconque des revendications 1 à 4, dans lequel l'étape d) comprend un traitement mécanique selon la revendication 5 à 9 et un traitement avec un ou plusieurs agents chimiques selon la revendication 11.

13. Procédé selon l'une quelconque des revendications 4 à 12, dans lequel la partie A est la partie mineure de la suspension concentrée prétraitée obtenue dans l'étape a, 1 à 20 % (poids en matière sèche).

14. Procédé selon l'une quelconque des revendications précédentes, dans lequel le micro-organisme est un champignon, qui est choisi dans le groupe constitué de : *Trichoderma sp., Aspergillus sp., Penicillium sp.* et *Talaromyces sp.*

15. Procédé selon la revendication 14, dans lequel le micro-organisme est *Trichoderma reesei.*
